# EUROPEAN PATENT SPECIFICATION

(11) **EP 2 080 745 B1**
(45) Date of publication and mention of the grant of the patent: **28.07.2010**
(21) Application number: 07025109.5
(22) Date of filing: 27.12.2007
(51) Int. Cl.: C07C 2/08, C08F 6/02, B01J 19/18

(54) **Method for oligomerisation of ethylene and reactor system therefore**
Verfahren zur Oligomerisierung von Ethylen und Reaktorsystem dafür
Procédé pour l'oligomérisation de l'éthylène et système de réacteur correspondant

(43) Date of publication of application: 22.07.2009
(73) Proprietor: Linde AG, 80331 München (DE); Saudi Basic Industries Corporation, 11422 Riyadh (SA)
(72) Inventor: Müller, Wolfgang, 81245 München (DE); Fritz, Peter M., 82008 Unterhaching (DE); Bölt, Heinz, 82515 Wolfratshausen (DE); Wellenofer, Anton, 81247 München (DE); Meiswinkel, Andreas, 81479 München (DE); Taube, Carsten, 85560 Ebersberg (DE)
(74) Representative: Scholz, Volker

(56) References cited:
- EP-A- 1 752 433
- EP-A- 1 754 694
- DE-A1- 19 807 226
- US-A- 3 074 921
- US-A- 5 522 553

## Description

The present invention relates to a method for the oligomerisation of ethylene and a reactor system therefore.

Oligomerisation methods for preparing linear alpha-olefins by oligomerisation of ethylene are widely known. These methods are usually carried out in a reactor system wherein ethylene introduced is converted by a suitable catalyst composition in presence of a solvent to linear alpha-olefins. After oligomerisation in the reactor gaseous and liquid outlet streams are further processed. The liquid outlet stream from the reactor usually contains linear alpha-olefins, solvent and the still active catalyst composition. One essential feature of the respective method is the deactivation of the components of the catalyst composition and the extraction from the organic phase. Usually, deactivation and extraction is achieved by mixing the liquid reactor outlet with a polar phase, e.g. an aqueous caustic solution. In this regard, a fast and effective mixing of the organic phase with the polar phase is required. Additionally, the formation of very small droplets and a minimum residence time of outlet stream and polar phase in the mixer (until effective mixing has been achieved) has to be ensured in order to create sufficient phase transfer surface. If the deactivation is not achieved fast enough, undesired side reactions will take place. This leads to a down grading of the product purities, and undesired by-products may form corrosive components in the separation section.

In the prior art static mixers, jet nozzles or stirred tank mixers have been utilized for mixing the organic outlet phase and the polar phase. However, these mixing devices have been so far insufficient to fulfil all the above requirements and can be further improved.

US 3,074,921 A discloses an apparatus for a continuous polymerization of ethylene, wherein a reactor is connected to a mixer where the active catalyst composition is quenched by mixing with an alcohol. The thus quenched outlet stream from the polymerization reactor is then transferred to a kettle for further thoroughly mixing of the alcohol and polymer-catalyst-mixture so as much of the catalyst as possible would be deactivated and solubilized in this step. Subsequently, the mixture is then transferred to an extraction column where the deactivated catalyst is extracted from the polymer.

It is therefore an object of the present invention to provide a method for the oligomerisation of ethylene which overcomes the drawbacks of the prior art. Especially a method shall be provided wherein after oligomerisation the catalyst composition may be easily and fast deactivated and extracted from the organic outlet stream of the oligomerisation reactor.

Additionally, it is an object to provide a reactor system for such an oligomerisation.

The first object is achieved by a method for the oligomerisation of ethylene, comprising the steps of:
(i) oligomerising ethylene in a reactor in the presence of a solvent and a catalyst composition;
(ii) discharging a catalyst composition containing outlet stream from the reactor;
(iii) deactivating and extracting the catalyst composition with a polar phase, wherein the outlet stream and the polar phase are mixed in a dynamic mixing device having rotor and stator elements comprising concentric tool rings that are radially slotted and/or drilled, wherein the annular shearing gap between adjacent tool rings is from 0.1 to 5 mm.

Preferably, the polar phase is an aqueous caustic solution.

More preferably, the rotational speed of the dynamic mixer is from 2.5 to 40 m/s.

In one embodiment, the catalyst composition comprises a zirconium salt of organic acids and at least one organo aluminum compound.

Further, it is preferred, that the zirconium salt has the formula ZrCl₄₋ₘXₘ, wherein X = OCOR or OSO₃R' with R and R' being independently alkyl, alkene or phenyl, and wherein 0 < m < 4.

Additionally, it is preferably proposed that at least one aluminum compound has the general formula R¹ₙAl₃₋ₙ or Al₂Y₃R¹₃, wherein R¹ represents an alkyl group having from 1 to 20 carbon atoms, Y represents Cl, Br or I, n is any number within the range 1 < n < 2.

According to the invention is also a reactor system for the oligomerisation of ethylene, comprising a reactor having inlets and outlets for feeding and discharging ethylene, alpha-olefins, solvent and catalyst compositions to and from the reactor, the reactor being connected with a dynamic mixing device where a catalyst composition containing outlet stream is mixed with a polar phase for deactivation and extraction of the catalyst, the dynamic mixing device having rotor and stator elements comprising concentric tool rings that are radially slotted and/or drilled, wherein the annular shearing gap is between 0.1 and 5 mm.

Preferably the dynamic mixing device includes cutting devices for treating of high molecular weight linear alpha-olefins up-streams of the stator and rotor elements or integrated into an inlet zone of the dynamic mixing device or in a separate casing.

Surprisingly it was found that the use of the specific dynamic mixing device as disclosed above results in a method for the oligomerisation of ethylene wherein a fast and effective mixing of liquid reactor outlet streams containing the catalyst composition with a polar phase for deactivation and extraction can be achieved. Additionally, very small droplets are formed and a minimum residence time until effective mixing has been performed is achieved. Further, the deactivation utilizing the dynamic mixing device is fast enough, so that undesired side reactions will be avoided. Thus, no downgrading of product purities is obtained, and a formation of corrosive components in the separation section is avoided.

By adapting the mixing device geometry as desired, the droplet size can be adjusted to fulfil the required fast and effective deactivation and extraction. On the other hand, the formation of a stable emulsion can be avoided so that the separation of the organic phase from the polar phase by gravity separation in a decanter can still be achieved afterwards.

In detail, the performance of the dynamic mixing device can be optimized by adaptation of the mixer geometry, like the gaps and distances between rotor and stator elements and the applied rotational speed.

It has been found that mixer types in which mixing shall be achieved by turbulence are not suitable for the purposes of the present invention, but high shear forces are needed to fulfil the required highly efficient mixing task. Satisfying results were only achieved utilizing the dynamic mixer as disclosed above. High shear forces and low residence time can then be realized.

The droplet size achieved is around 10 µm with a narrow size distribution.

In a further embodiment, the mixing device may include cutting devices for treating of high molecular weight linear alpha-olefins upstreams of the mixing elements or integrated into an inlet zone of the dynamic mixing device or in a separate casing. This assists in avoiding deposition and plugging of the reactor system.

Further, dead zones within the dynamic mixing device can be significantly avoided which also adds to the benefits of the inventive method and reactor system.

The features disclosed in the foregoing description, or in the claims may, both separately and in any combination thereof, be material for realizing the invention in diverse forms thereof.

## Claims

1. Method for the oligomerisation of ethylene, comprising the steps of:
(i) oligomerising ethylene in a reactor in the presence of a solvent and a catalyst composition;
(ii) discharging a catalyst composition containing outlet stream from the reactor;
(iii) deactivating and extracting the catalyst composition with a polar phase, wherein the outlet stream and the polar phase are mixed in a dynamic mixing device having rotor and stator elements comprising concentric tool rings, that are radially slotted and/or drilled, wherein the annular shearing gap is from 0.1 to 5 mm

2. Method according to claim 1, wherein the poplar phase is an aqueous caustic solution.

3. Method according to claim 1 or 2, wherein the rotational speed of the dynamic mixing device is from 2.5 to 40 m/s.

4. Method according to any of the preceding claims, wherein the catalyst composition comprises a zirconium salt of organic acids and at least one organo aluminum compound.

5. Method according to claim 4, wherein the zirconium salt has the formula ZrCl₄₋ₘXₘ, wherein X = OCOR or OSO₃R' with R and R' being independently alkyl, alkene or phenyl, and wherein 0 < m < 4.

6. Method according to claim 4 or 5, wherein the at least one aluminum compound has the general formula R¹ₙAl₃₋ₙ or Al₂Y₃R¹₃, wherein R¹ represents an alkyl group having from 1 to 20 carbon atoms, Y represents Cl, Br or I, n is any number within the range 1 < n < 2.

7. Reactor system for the oligomerisation of ethylene, comprising a reactor having inlets and outlets for feeding and discharging ethylene, alpha-olefins, solvent and catalyst composition to and from the reactor, the reactor being connected with a dynamic mixing device where a catalyst composition containing outlet stream is mixed with a polar phase for deactivation and extraction of the catalyst, the dynamic mixing device having rotor and stator elements comprising concentric tool rings, that are radially slotted and/or drilled, wherein the annular shearing gap is from 0.1 to 5 mm.

8. Reactor system according to claim 7, wherein the dynamic mixing device includes cutting devices for treating of high molecular weight linear alpha-olefins upstreams of the stator and rotor elements, or integrated into an inlet zone of the dynamic mixing device, or in a separate casing.

## Patentansprüche

1. Verfahren zur Oligomerisation von Ethylen, welches die Schritte umfasst:
(i) Oligomerisieren von Ethylen in einem Reaktor in der Gegenwart eines Lösungsmittels und einer Katalysatorzusammensetzung;
(ii) Abgeben eines Katalysatorzusammensetzung enthaltenden Auslassstroms aus dem Reaktor;
(iii) Deaktivieren und Extrahieren der Katalysatorzusammensetzung mit einer polaren Phase, wobei der Auslassstrom und die polare Phase gemischt werden in einer dynamischen Mischvorrichtung mit Rotor- und Statorelementen umfassend konzentrische Werkzeugringe, die radial geschlitzt und/oder gebohrt sind, wobei der ringförmige Scherungsabstand von 0,1 bis 5 mm ist.

2. Verfahren nach Anspruch 1, wobei die polare Phase eine wässerige Alkalilösung ist.

3. Verfahren nach Anspruch 1 oder 2, wobei die Drehgeschwindigkeit der dynamischen Mischvorrichtung von 2,5 bis 40 m/s ist.

4. Verfahren nach einem der vorangehenden Ansprüche, wobei die Katalysatorzusammensetzung ein Zirkoniumsalz organischer Säuren und wenigstens eine Organoaluminumverbindung umfasst.

5. Verfahren nach Anspruch 4, wobei das Zirkoniumsalz die Formel ZrCl₄₋ₘXₘ aufweist, wobei X = OCOR oder OSO₃R' ist, wobei R und R' unabhängig Alkyl, Alken oder Phenyl sind, und wobei 0 < m < 4 ist.

6. Verfahren nach Anspruch 4 oder 5, wobei die wenigstens eine Aluminumverbindung die allgemeine Formel R¹ₙAl₃₋ₙ oder Al₂Y₃R¹₃ aufweist, wobei R¹ eine Alkylgruppe mit 1 bis 20 Kohlenstoffatomen darstellt, Y Cl, Br oder I darstellt, n irgendeine Zahl im Bereich von 1 < n < 2 ist.

7. Reaktorsystem zur Oligomerisation von Ethylen umfassend einen Reaktor mit Einlässen und Auslässen zum Zuführen und Abführen von Ethylen, alpha-Olefinen, Lösungsmittel und Katalysatorzusammensetzung zu und von dem Reaktor, wobei der Reaktor mit einer dynamischen Mischvorrichtung verbunden ist, wo ein Katalysatorzusammensetzung enthaltender Auslassstrom mit einer polaren Phase zur Deaktivierung und Extraktion des Katalysators gemischt wird, wobei die dynamische Mischvorrichtung Rotor- und Statorelemente umfassend konzentrische Werkzeugringe aufweist, die radial geschlitzt und/oder gebohrt sind, wobei der ringförmige Scherungsabstand von 0,1 bis 5 mm ist.

8. Reaktorsystem nach Anspruch 7, wobei die dynamische Mischvorrichtung Schneidvorrichtungen zum Behandeln der hochmolekulargewichtigen linearen alpha-Olefine stromaufwärts der Stator- und Rotorelemente oder integriert in eine Einlasszone der dynamischen Mischvorrichtung oder in einem getrennten Gehäuse einschließt.

## Revendications

1. Procédé d'oligomérisation d'éthylène comprenant les étapes consistant à :
(i) oligomériser l'éthylène dans un réacteur en présence d'un solvant et d'une composition catalytique ;
(ii) évacuer un flux de sortie contenant la composition catalytique du réacteur ;
(iii) désactiver et extraire la composition catalytique avec une phase polaire, le flux de sortie et la phase polaire étant mélangés dans un dispositif de mélange dynamique comprenant des éléments rotors et stators comprenant des anneaux porte-outil concentriques qui sont radialement insérés et/ou forés, l'espace de cisaillement annulaire étant de 0,1 à 5 mm.

2. Procédé selon la revendication 1, dans lequel la phase polaire est une solution caustique aqueuse.

3. Procédé selon la revendication 1 ou 2, dans lequel la vitesse rotationnelle du dispositif de mélange dynamique est de 2,5 à 40 m/s.

4. Procédé selon l'une quelconque des revendications précédentes, dans lequel la composition catalytique comprend un sel de zirconium d'acides organiques et au moins un composé à base d'organoaluminium.

5. Procédé selon la revendication 4, dans lequel le sel de zirconium a la formule ZrCl₄₋ₘXₘ, dans laquelle X = OCOR ou OSO₃R', R et R' étant indépendamment un groupe alkyle, alcène ou phényle, et dans laquelle 0 < m < 4.

6. Procédé selon la revendication 4 ou 5, dans lequel le ou les composés à base d'aluminium ont la formule générale R¹ₙAl₃₋ₙ ou Al₂Y₃R¹₃, dans laquelle R¹ représente un groupe alkyle ayant de 1 à 20 atomes de carbone, Y représente Cl, Br ou I, n est n'importe quel nombre compris dans la plage 1 < n < 2.

7. Système de réacteur pour l'oligomérisation de l'éthylène comprenant un réacteur ayant des entrées et des sorties pour l'alimentation et l'évacuation de l'éthylène, des alpha-oléfines, du solvant et de la composition catalytique dans et hors du réacteur, le réacteur étant relié à un dispositif de mélange dynamique dans lequel un flux de sortie contenant la composition catalytique est mélangé avec une phase polaire pour la désactivation et l'extraction du catalyseur, le dispositif de mélange dynamique ayant des éléments rotors et stators comprenant des anneaux porte-outil concentriques qui sont radialement insérés et/ou forés, l'espace de cisaillement annulaire étant de 0,1 à 5 mm.

8. Système de réacteur selon la revendication 7, dans lequel le dispositif de mélange dynamique comprend des dispositifs de coupe pour traiter les alpha-oléfines linéaires de poids moléculaire élevé en amont des éléments stators et rotors, ou intégrés dans une zone d'entrée du dispositif de mélange dynamique ou dans un logement séparé.
